(19) Europäisches Patentamt
European Patent Office
Office européen des brevets

(11) **EP 4 014 950 A1**

(12) **EUROPÄISCHE PATENTANMELDUNG**

(43) Veröffentlichungstag:
**22.06.2022 Patentblatt 2022/25**

(21) Anmeldenummer: **20215553.7**

(22) Anmeldetag: **18.12.2020**

(51) Internationale Patentklassifikation (IPC):
*A61K 6/84* (2020.01)    *C22C 1/02* (2006.01)
*C22C 38/00* (2006.01)   *C22C 38/02* (2006.01)
*C22C 38/22* (2006.01)   *C22C 38/26* (2006.01)
*C22C 38/38* (2006.01)

(52) Gemeinsame Patentklassifikation (CPC):
**C22C 38/001; A61K 6/844; C22C 1/02;
C22C 38/002; C22C 38/02; C22C 38/22;
C22C 38/26; C22C 38/38;** C21D 2211/001

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Deutsche Edelstahlwerke
Specialty Steel GmbH & Co. KG
58452 Witten (DE)**

(72) Erfinder:
• **Dr. Mohr, Andreas
45131 Essen (DE)**

• **Dr. Hill, Horst
47929 Grefrath (DE)**
• **Ripkens, Oliver
47647 Kerken (DE)**
• **Dr. Niederhofer, Philipp
44803 Bochum (DE)**
• **Conrads, Janosch
45968 Gladbeck (DE)**

(74) Vertreter: **Cohausz & Florack
Patent- & Rechtsanwälte
Partnerschaftsgesellschaft mbB
Bleichstraße 14
40211 Düsseldorf (DE)**

(54) **STAHL MIT EINEM AUSTENITISCHEN GEFÜGE UND VERWENDUNG EINES SOLCHEN STAHLS FÜR ANWENDUNGEN IM MUNDRAUM EINES MENSCHEN ODER EINES TIERS**

(57)   Die Erfindung stellt einen nickel- und kobaltfreien, nichtrostenden austenitischen Stahl zur Verfügung, der die in der Dentaltechnik benötigten Anforderungen an seine Korrosionsbeständigkeit, seinen Wärmeausdehnungskoeffizienten, seine mechanischen Eigenschaften und seine Gießbarkeit erfüllt. Der erfindungsgemäße Stahl besteht hierzu aus, in Masse-%, C: 0,05 - 0,8 %,N: 0,3 - 1,3 %,Si: < 2,0 %,Mn: 14 - 30 %,Cr: 17 - 27 %,Mo: 0 - 6 %,W: 0 - 6 %,Nb: 0 - 6 %,Ga: 0 - 6 %,Ta 0 - 6 %, Rest Eisen und unvermeidbaren Verunreinigungen, wobei der Gehalt an Verunreinigungen auf weniger als 0,5 % beschränkt ist und zu den Verunreinigungen weniger als 0,1 % Ni und weniger als 0,1 % Co zählen. Der erfindungsgemäß zusammengesetzte Stahl weist ein austenitisches Gefüge, eine Dehngrenze Rp0,2 von mindestens 230 MPa, eine Bruchdehnung A von mehr als 5 % und mit ein E-Modul von mindestens 150 GPa auf.

EP 4 014 950 A1

**Beschreibung**

Die Erfindung betrifft einen Stahl mit einem austenitischen Gefüge.

Ebenso betrifft die Erfindung die Verwendung eines Stahls insbesondere für dentaltechnische Anwendungen.

Die im vorliegenden Text angegebenen mechanischen Kennwerte und Korrosionseigenschaften sind gemäß DIN EN ISO 22674 (hier ab Festigkeitsklasse 3 dargestellt) ermittelt.

Wenn im vorliegenden Text "%"-Angaben zu Legierungen oder Stahlzusammensetzungen gemacht werden, so beziehen diese sich jeweils auf die Masse (Angabe in "Masse=%"), soweit nicht ausdrücklich etwas anderes genannt ist.

Die heute üblicherweise verwendeten metallischen Dentallegierungen können in zwei Bereiche unterteilt werden. Zum einen Bereich zählen die Edelmetalllegierungen, zu denen Werkstoffe auf Basis von Gold, Silber, Palladium oder Platin gehören. Dem anderen Bereich werden die kostengünstigeren Nichtedelmetalllegierungen zugerechnet. Diese können wiederum aufgrund ihrer Hauptlegierungssysteme Nickel-Chrom (NiCr) und Kobalt-Chrom (CoCr) unterschieden werden.

Derzeit sind in Deutschland etwa 1400 unterschiedliche Dentallegierungen zugelassen. Sie zeichnen sich durch eine sehr gute Korrosionsbeständigkeit aus, um korrosiven Angriff durch Speichel, Magensäure und anderen im Mundraum auftretenden Medien zu widerstehen. Zudem müssen Werkstoff für dentaltechnische Anwendungen eine gewisse Härte und mechanische Festigkeit aufweisen, um die beim Kauen auftretenden Belastungen ertragen zu können.

Zusätzlich müssen Dentallegierungen amagnetisch und biokompatibel sein, um Abstoßungsreaktionen, allergische Reaktionen oder sonstige Sensibilisierungen des Patienten zu vermeiden. Diese Anforderungen erweisen sich insbesondere bei Werkstoffen auf NiCr-Basis als Problem. Eine Nickelallergie ist die am häufigsten auftretende allergische Reaktion auf einen metallischen Werkstoff. NiCr-Legierungen lassen sich daher nur im eingeschränkten Maße verwenden.

Als Alternative zu den NiCr-Basiswerkstoffen werden insbesondere in Europa CoCr-Legierungen für medizinischen Zahnersatz eingesetzt. Jedoch sind diese deutlich teurer als Nickellegierungen. Zudem besteht der Verdacht, dass Kobalt im Körper Krebs auslösen bzw. begünstigen könnte. Sollte sich dieser Verdacht bestätigen, so werden voraussichtlich auch Kobaltlegierungen in der Zukunft nicht mehr im menschlichen Körper eingesetzt werden können.

Ein weiteres Beispiel für einen Stahl, der besonders für die Anwendung am menschlichen Körper vorgesehen ist, ist aus der EP 0 918 099 A1 bekannt. Hierbei handelt es sich um eine Chrom-Mangan-Stahllegierung, die aus, in Masse-%, 0,08 - 0,25 % C, $\leq$ 0,015 % S, $\leq$ 0,050 % P, 12 - 17 % Mn, 0,2 - 1 % Si, 1 - 3 % Cu, 2-6 % Co, $\leq$ 0,01 % Titan, 3 - 6 % Mo, 17 - 22 % Cr, $\leq$ 1,0 % Ni, $\leq$ 0,01 % Al, $\leq$ 0,01 % Niob, $\leq$ 0,01 % B, $\leq$ 0,2 % V, 0,5 - 0,9 % N und als Rest aus Eisen und erschmelzungsbedingten Verunreinigungen besteht, wobei der Stahl ein Nickel-Äquivalent über 17, vorzugsweise über 20, und einen gemäß der Formel PREN = %Cr + 3,3 x %Mo + 20 x %N aus dem Cr-Gehalt %Cr, dem Mo-Gehalt %Mo und dem N-Gehalt %N berechneten, für eine gute Korrosionsbeständigkeit stehenden PREN-Wert von mindestens 37, insbesondere mehr als 45, aufweisen soll.

Neben den Reaktionen, die Nickel- und Kobaltlegierungen im menschlichen Körper auslösen können, müssen auch die arbeitsschutztechnischen Aspekte bei der Verarbeitung dieser Legierungen berücksichtigt werden. Die Verarbeitung von Dentalwerkstoffen erfolgt zumeist in Handarbeit durch spanabhebende Bearbeitung, wie durch Schleifen, Fräsen und desgleichen. Hieraus ergibt sich die Gefahr, dass entstehende Stäube über die Lunge in den Körper gelangen und ihn auf diesem Weg schädigen können.

Vor diesem Hintergrund hat sich der Bedarf nach Legierungen für die Anwendung im Mundraum ergeben, welche frei von Ni und Co sind, jedoch ähnliche Eigenschaften wie die bekannten NiCr- und CoCr-Legierungen aufweisen. So sollen Dentallegierungen der hier in Rede stehenden Art

-  ein austenitisches und damit einhergehend amagnetisches Gefüge,

-  eine hohe Korrosionsbeständigkeit, die durch eine Freisetzung von weniger als 200 $\mu$g/cm$^2$ innerhalb von 7 Tagen gekennzeichnet ist,

-  eine Dehngrenze Rp0,2 von mindestens 270 MPa,

-  eine Bruchdehnung A von mehr als 5 %,

-  ein E-Modul von mehr als 150 GPa (erst ab der Festigkeitsklasse 5 definiert),

-  eine gute Vergießbarkeit,

-  eine gute mechanische Bearbeitbarkeit

und

- eine gute Eignung zur keramischen Verblendung

aufweisen.

Die Eignung zur keramischen Verblendung setzt voraus, dass es weder bei der Herstellung, noch beim Gebrauch zu thermisch bedingten Spannungen zwischen dem aus der jeweiligen Legierung bestehenden Bauelement und der Keramikschicht kommt, mit der das Bauteil verblendet ist. Dies kann dadurch gesichert werden, dass der Wärmeausdehnungskoeffizient der Legierung des Bauteils an den Wärmeausdehnungskoeffizient der Verblendungskeramik angepasst werden kann. Alternativ oder ergänzend ist es auch möglich, ein Abplatzen der Verblendung dadurch zu verhindern, dass die Anbindung zwischen dem Metallwerkstoffs des Bauteils und der Verblendungskeramik reaktive Elemente verbessert werden. Die hierzu geeigneten Elemente weisen allerdings in der Regel eine hohe Affinität zu Sauerstoff auf, wie z.B. Mangan.

Vor dem Hintergrund des voranstehend erläuterten Standes der Technik hat sich die Aufgabe ergeben, einen nickel- und kobaltfreien, nichtrostenden austenitischen Stahl zu entwickeln, der die in der Dentaltechnik benötigten Anforderungen an seine Korrosionsbeständigkeit, seine mechanischen Eigenschaften, seine Gießbarkeit und seinen Wärmeausdehnungskoeffizienten zuverlässig erfüllt.

Die Erfindung hat diese Aufgabe durch einen Stahl gelöst, der die in Anspruch 1 angegebene Zusammensetzung besitzt und aufgrund seines Eigenschaftsspektrums im Besonderen für die Herstellung von Bauteilen zur Verwendung im Mundraum von Menschen oder Tieren geeignet ist.

Vorteilhafte Ausgestaltungen der Erfindung sind in den abhängigen Ansprüchen angegeben und werden wie der allgemeine Erfindungsgedanke nachfolgend im Einzelnen erläutert.

Ein erfindungsgemäßer Stahl besitzt demnach ein austenitisches, amagnetisches Gefüge und weist eine Dehngrenze $Rp_{0,2}$ von mindestens 230 MPa, eine Bruchdehnung A von mehr als 5 % und einen E-Modul von mindestens 150 GPa auf. Hierzu besteht der Stahl erfindungsgemäß aus, in Masse-%,

| C: | 0,05 | - | 0,8 %, |
|---|---|---|---|
| N: | 0,3 | - | 1,3 %, |
| Si: | | < | 2,0 %, |
| Mn: | 14 | - | 30 %, |
| Cr: | 17 | - | 27 %, |
| Mo: | 0 | - | 6 %, |
| Nb: | 0 | - | 6 %, |
| W: | 0 | - | 6 %, |
| Ga: | 0 | - | 6 %, |
| Ta | 0 | - | 6 %, |

Rest Eisen und unvermeidbaren Verunreinigungen, wobei der Gehalt an Verunreinigungen auf weniger als 0,5 % beschränkt ist und zu den Verunreinigungen < 0,1 % Ni und < 0,1 % Co zählen.

Die Erfindung ist ausgegangen von der Erkenntnis, dass es mit höheren Gehalten an Kohlenstoff ("C") und Stickstoff ("N") zuverlässig gelingt, austenitisch erstarrende, nichtrostende Stahlwerkstoffe zu erzeugen, wie beispielsweise aus der DE 10146616 A1 bekannt. Solche Stahlwerkstoffe besitzen in Summe 0,4 - 0,7 Masse-% C und N, bei bis zu 0,7 Masse-% Si, 17 - 21 Masse-% Mn und 16,5 - 18,5 Masse-% Cr, Rest Eisen, einen hohen Anteil an den interstitiell gelösten C- und N-Atomen. Diese stabilisieren das austenitische Gefüge und verbessern die mechanischen Eigenschaften der Legierung durch eine erhöhte Mischkristallverfestigung. So weisen C+N-legierte, nichtrostende Austenite deutlich höhere Festigkeiten auf als die klassischen nichtrostenden austenitischen Stähle, wie z.B. der unter der Werkstoffnummer 1.4404 genormte Stahl, der aus, in Masse-%, bis zu 0,03 % C, bis zu 1,00 % Si, bis zu 2,00 % Mn, 16,5 - 18,5 % Cr, 2,0 - 2,5 Mo und 10,0 - 13,0 % Ni, Rest Eisen und unvermeidbaren Verunreinigungen besteht.

Um günstige Effekte der Anwesenheit von C und N ebenfalls zu nutzen und auszubauen, sieht die Erfindung einen C-Gehalt von 0,05 Masse-% bis 0,8 Masse-%, insbesondere bis 0,7 Masse-%, und einen N-Gehalt von 0,3 Masse-% bis 1,3 % Masse-%, insbesondere bis 1,0 Masse-%, vor, so dass die Summe der Gehalte an C und N bei einem erfindungsgemäßen Stahl 0,35 Masse-% bis 2,1 Masse-%, insbesondere bis 2,0 Masse-%, bis 1,8 Masse-% oder bis 1,7 Masse-%, beträgt. Die erfindungsgemäß vorgesehenen hohen C- und N-Gehalte führen dazu, dass C und N einen weiter vergrößerten Anteil an der Mischkristallverfestigung des erfindungsgemäßen Stahls und damit zu seinen hohen mechanischen Kennwerten leistet. Darüber hinaus besitzt der erfindungsgemäße C+N-Stahl eine weiter erhöhte Korrosionsbeständigkeit, insbesondere gegen Lochkorrosion, durch seinen hohen Anteil an interstitiell gelöstem N. Gleichzeitig wird durch die erfindungsgemäß vorgesehenen Erweiterungen der Gehalte an C und N ein größeres Schmelzintervall eröffnet und damit einhergehend die schmelzmetallurgische Verarbeitung durch ein besseres Gießverhalten einer erfindungsgemäß legierten Schmelze erleichtert. Durch die erfindungsgemäß vorgeschriebenen Mindestgehalte an C und N ist sichergestellt, dass diese Effekte sicher eintreten. Dagegen ist der C- und N-Gehalt eines erfindungsgemäßen Stahls auf in Summe höchstens 2,1 Masse-% beschränkt, um eine verstärkte Karbid- oder Nitridbildung zu vermeiden. Diese würde die Korrosionseigenschaften senken. Gleichzeitig würden höhere C- und N-Gehalte zwar die Festigkeiten und Härte steigern. Jedoch würde dies die Ver- und Nachbearbeitung des Werkstoffs stark erschweren. Hinzukommt bei einem zu hohen C- und N-Gehalt ein starker Abfall der Zähigkeit. Hinsichtlich einer optimierten Wirkung von C und N haben sich C- und N-Gehalte von in Summe höchstens 2,0 Masse-% (bei einem C-Gehalt von höchstens 0,7 Masse-% und einem N-Gehalt von höchstens 1,3 Masse-%), von in Summe höchstens 1,8 Masse-% (bei einem C-Gehalt von höchstens 0,8 Masse-% und einem N-Gehalt von höchstens 1,0 Masse-%) oder von in Summe höchstens 1,7 Masse-% (bei einem C-Gehalt von höchstens 0,7 Masse-% und einem N-Gehalt von höchstens 1,0 Masse-%) besonders bewährt.

Um zu vermeiden, dass bei einem zu hohen C-Angebot andere Legierungselemente, wie W, Mo und Cr, primäre Karbide bilden, kann der C-Gehalt auf einem im Vergleich zum N-Gehalt niedrigen Niveau gehalten werden. Derartige primäre Karbide sind sowohl bei der schmelzmetallurgischen Herstellung von aus erfindungsgemäßem Stahl bestehenden Halbzeugen, als auch bei der späteren Verarbeitung im Dentallabor als kritisch zu sehen, da sie sich aus der Schmelze bilden und zu einer reduzierten Korrosionsbeständigkeit und zur Versprödung führen können. Aus diesem Grund kann es sinnvoll sein, den C-Gehalt auf Werte von höchstens 0,7 Masse-%, insbesondere höchstens 0,6 Masse-%, zu beschränken und die Austenitstabilität primär durch die Elemente Mn und N zu erreichen. Hierzu haben sich N-Gehalte von mindestens 0,4 Masse-%, insbesondere mindestens 0,5 Masse-%, besonders bewährt.

Silizium ("Si") kann im erfindungsgemäßen Stahl in Gehalten von bis zu 2,0 Masse-% vorgesehen sein. Bei höheren Si-Gehalten würde im Gefüge des Stahls zu viel Ferrit stabilisiert mit der Folge, dass der Werkstoff seine amagnetischen Eigenschaften verlieren würde. Negative Auswirkungen der Anwesenheit von Si können dadurch vermieden werden, dass der Si-Gehalt auf höchstens 1,5 Masse-%, insbesondere höchstens 1,3 Masse-%, beschränkt wird.

Wie schon erwähnt, ist Mangan ("Mn") im erfindungsgemäßen Stahl in Gehalten von 14 Masse-% bis 30 Masse-%, insbesondere 16 Masse-% bis 28 Masse-%, vorhanden, um die Zulegierung der erfindungsgemäß vorgesehenen hohen N-Gehalte durch die in Folge der Anwesenheit von Mn erhöhten Löslichkeit der Schmelze zu erleichtern. Dadurch ist die Herstellung der Stähle unter Atmosphärendruck möglich. Zudem ist Mn, wie C und N, ein starker Austenitstabilisator.

Durch die kombinierte Anwesenheit der erfindungsgemäß vorgegebenen Gehalte an Mn, C und N gelingt es so, Nickel ("Ni") als Austenitstabilisator vollständig zu substituieren. Besonders effektiv lassen sich die positiven Einflüsse von Mn auf die Eigenschaften eines erfindungsgemäß legierten Stahls bei Mn-Gehalten von mindestens 16 Masse-%, insbesondere mindestens 17 Masse-%, nutzen. Bei oberhalb von 30 Masse-% liegenden Mn-Gehalten besteht allerdings die Gefahr, dass mit Blick auf die amagnetischen Eigenschaften unerwünschter Ferrit im Gefüge des erfindungsgemäßen Stahls gebildet wird. Zudem würden zu hohe Mn-Gehalte den Wärmeausdehnungskoeffizienten erhöhen. Auch diese Wirkung sollte mit Blick auf den praktischen Nutzen des erfindungsgemäßen Werkstoffs vermieden werden.

Der hohe Mn-Gehalt eines erfindungsgemäßen Stahls hat in Kombination mit den ebenfalls vorgeschriebenen Cr-Gehalten den weiteren Effekt, dass der erfindungsgemäße Stahl eine hohe Affinität zu Sauerstoff hat. Die in der Dentalindustrie verwendeten Keramiken sind fast ausschließlich Oxidkeramiken, wodurch in den Verblendungskeramiken ein hoher Anteil Sauerstoff vorhanden ist. Durch die Variation des Mangangehaltes im Rahmen der erfindungsgemäßen Maßgabe kann somit die Haftung zwischen Legierung und Verblendungskeramik unmittelbar beeinflusst werden. Hierzu haben sich Mn-Gehalte des erfindungsgemäßen Stahls von höchstens 28 Masse-%, insbesondere höchstens 27 Masse-%, besonders bewährt.

In Folge der erfindungsgemäß vorgenommenen Substitution von Ni durch C, N und Mn kann der Gehalt an Nickel ("Ni") im erfindungsgemäßen Stahl so weit reduziert werden, dass er im technischen Sinne "0 Masse-%" ist, jedenfalls aber kleiner 0,1 Masse-%, und damit hinsichtlich der hier im Mittelpunkt stehenden Eigenschaften des erfindungsgemäßen Stahls unwirksam ist. Eine potenziell allergene Wirkung geht bei diesen Ni-Gehalten vom erfindungsgemäßen Stahl sicher nicht mehr aus. Allenfalls ist Ni somit, soweit überhaupt vorhanden, den unvermeidbaren Verunreinigungen zuzurechnen, die im Stahl nicht gezielt zugegeben werden, jedoch im Zuge der Stahlerzeugung in den Stahl gelangen können.

Genauso ist der Gehalt an Kobalt ("Co") im erfindungsgemäßen Stahl auf Werte von weniger als 0,1 Masse-% reduziert, bei denen Co keine Wirkung mehr auf die Eigenschaften des erfindungsgemäßen Stahlwerkstoffs hat und keine gesundheitsgefährdende Wirkung von seiner Anwesenheit ausgehen kann. Dementsprechend ist der Co-Gehalt erfindungsgemäß ebenfalls bevorzugt auf im technischen Sinne "0 Masse-%" reduziert, jedenfalls aber so gering, dass er allenfalls in den Gehalten vorliegt, die den herstellungsbedingt unvermeidbaren Verunreinigungen zuzurechnen sind.

Chrom ("Cr") ist im erfindungsgemäßen Stahl in Gehalten von 17 Masse-% bis 27 Masse-% vorhanden, um eine ausreichende Korrosionsbeständigkeit zu gewährleisten. Cr bildet in diesem Gehaltsbereich eine dichte Chromoxidschicht auf aus erfindungsgemäßem Stahl gefertigten Bauelement, welche Korrosionsreaktionen unterbindet. Durch Gehalte von mindestens 17 Masse-% ist sichergestellt, dass die Korrosionsbeständigkeit auch in dem potenziell sehr aggressiven Umfeld gegeben ist, das sich in der Mundhöhle ergeben kann. Durch eine Erhöhung des Cr-Gehalts auf mindestens 18 Masse-%, insbesondere mindestens 19 Masse-%, kann die Sicherheit, mit der der erfindungsgemäße Stahlwerkstoff korrosiven Angriffen widersteht, noch gesteigert werden. Nach oben ist der Cr-Gehalt eines erfindungsgemäßen Stahls auf höchstens 26 Masse-% beschränkt, um die übermäßige Bildung von primären Karbiden zu vermeiden, die die Zähigkeit und Dehnbarkeit des Stahls beeinträchtigen würden. Zudem würden Gehalte von mehr als 26 Masse-% Cr die ferritische Phase im Gefüge des erfindungsgemäßen Stahls stabilisieren mit der Folge, dass die unmagnetischen Eigenschaften des erfindungsgemäßen Stahlwerkstoffs nicht mehr gewährleistet wären. Den aufgezeigten negativen Auswirkungen der Anwesenheit von Cr im erfindungsgemäßen Stahl kann durch eine Beschränkung des Cr-Gehalts auf höchstens 25 Masse-%, insbesondere höchstens 24 Masse-%, besonders sicher begegnet werden.

Die Pitting Resistance Equivalent Number "PREN" drückt den Einfluss der Legierungselemente Cr, Mo und N auf die Korrosionsbeständigkeit des erfindungsgemäßen Stahlwerkstoffs aus, wobei die Erfindung den PREN-Wert gemäß der Gleichung PREN = %Cr + 3,3 x %Mo + 20 x %N, mit %Cr = jeweiliger Cr-Gehalt, %Mo = jeweiliger Mo-Gehalt und %N = jeweiliger N-Gehalt des Stahls, berechnet. Indem die Cr-, Mo- und N-Gehalte des erfindungsgemäßen Stahls so abgestimmt werden, dass sich PREN-Werte von mehr als 30%, insbesondere mehr als 34 %, ergeben, lässt sich eine optimierte Korrosionsbeständigkeit sichern, wobei PREN-Werte von mindestens 38 %, insbesondere mindestens 40 % oder mindestens 45 %, sich hier als besonders vorteilhaft erweisen.

Beim erfindungsgemäßen Stahlwerkstoff ist das Schmelzintervall, d.h. der Temperaturbereich, in dem der Stahl schmelzflüssig vorliegt, in einen günstigen Temperaturbereich verschoben. So beträgt er typischerweise Tsol=1250°C bis Tliq=1350 °C. Das somit gegenüber konventionellen Stählen der hier in Rede stehenden Gattung deutlich größere und schon bei niedrigeren Temperaturen zur Verfügung stehende Schmelzintervall, führt bei der schmelztechnischen Verarbeitung (Gießen) zu einer verbesserten Formfüllung bei gleichzeitig geringerer Belastung der Schmelzanlagen und Formmaterialen.

Die durch Cr auf dem erfindungsgemäßen Stahl gebildete Chromoxidschicht kann durch die Zugabe von bis zu 6 Masse-% Molybdän ("Mo") stabilisiert werden. Insbesondere kann durch die Zugabe von Mo eine Erhöhung der Beständigkeit gegen Lochkorrosion erzielt werden. Sollen sie genutzt werden, so kann hierzu ein Mo-Gehalt von mindestens 0,5 Masse-%, insbesondere mindestens 0,6 Masse-%, dem erfindungsgemäßen Stahl zugegeben werden. Besonders effektiv lassen sich die positiven Einflüsse von Mo bei Mo-Gehalten von bis zu 5,5 Masse-%, insbesondere bis zu 5,0 Masse-%, nutzen.

Wie Mo kann auch Wolfram ("W") zur Steigerung der Korrosionsbeständigkeit dem erfindungsgemäßen Stahl in Gehalten von bis zu 6 Masse-% zugegeben werden. Soll dieser Effekt genutzt werden, so kann hierzu ein W-Gehalt von mindestens 0,5 Masse-%, insbesondere mindestens 0,6 Masse-%, im erfindungsgemäßen Stahl vorgesehen werden. Besonders effektiv lassen sich die günstigen Auswirkungen der Anwesenheit von W bei W-Gehalten von bis zu 5,5 Masse-%, insbesondere bis zu 5,0 Masse-%, nutzen.

Eine weitere von der Erfindung gemeisterte Herausforderung bestand darin, die Legierung des erfindungsgemäßen Stahls so einzustellen, dass der Wärmeausdehnungskoeffizient WAK von Raumtemperatur ("RT") bis 400 °C auf ein Niveau reduziert ist, bei dem eine dauerhaft sichere Anhaftung einer auf den Stahl aufgebrachten keramischen Verblendung gesichert ist. Insbesondere aufgrund ihrer guten Löslichkeit im Fe bieten sich die Elemente Cr, Mo und W für die Senkung des Wärmeausdehnungskoeffizienten WAK an. Eine zu diesem Zweck vorgenommene Erhöhung der Gehalte an Cr, Mo und W alleine wäre jedoch mit einer verstärkten ferritstabilisierenden Wirkung einhergegangen. Diesem Effekt ist durch eine gleichzeitige Erhöhung der Gehalte an Elemente C, N und Mn entgegengewirkt worden. Im Ergebnis ist es so gelungen, den Wärmeausdehnungskoeffizienten WAK eines erfindungsgemäßen Werkstoffs auf Werte einzustellen, die höchstens 24 x 10$^{-6}$/K betragen, wobei der Wärmeausdehnungskoeffizient WAK in der Praxis typischerweise im Bereich von 15 x 10$^{-6}$/K bis 22 x 10$^{-6}$/K liegt.

Niob ("Nb") und/oder Gallium ("Ga") und/oder Tantal ("Ta") in Gehalten von jeweils bis zu 6 Masse-% können, gegebenenfalls alternativ zu oder in Kombination mit den hierzu vorgesehenen Mo- und/oder W-Gehalten, ebenfalls zur Absenkung des Wärmeausdehnungskoeffizienten dem Stahl zugegeben werden. Dieser Effekt kann bei Nb-Gehalten von mindestens 0,5 Masse-% und/oder Ga-Gehalten von mindestens 0,5 Masse-% und/oder Ta-Gehalten von mindestens 0,5 Masse-% sicher genutzt werden. Nb, Ta und Ga können dabei jeweils separat oder in Kombination zugegeben werden. Bei ausreichenden Gehalten an Mo kann jedoch auch vollständig auf die Zugabe von Nb, Ta und/oder Ga verzichtet werden. Ebenso kann jedes der Elemente Nb, Ta und Ga auch alleine zugegeben werden, um die durch die Anwesenheit dieser Elemente im erfindungsgemäßen Stahl ermöglichten Verbesserungen zu erzielen. Die positiven Effekte der optionalen Anwesenheit von Nb, Ta und/oder Ga lassen sich bei einem Gehalt von mindestens 0,6 Masse-% an Nb, Ta und/oder Ga besonders sicher nutzen, wobei Gehalte von mindestens 0,7 Masse-% an mindestens einem der Elemente Nb, Ta, Ga hierzu besonders zweckmäßig sein können.

Optionale Gehalte von jeweils bis zu 5,0 Masse-%, insbesondere bis zu 1,0 Masse-%, an Nb, Ta und/oder Ga haben sich als besonders praxisgerecht herausgestellt.

Im Fall, dass mehr als ein Element aus der Gruppe "Nb, Mo, W, Ga, Ta" vorhanden ist, lässt sich die Wirkung dieser Elemente insbesondere dann mit den voranstehend erläuterten Vorteilen nutzen, wenn die Gehalte an diesen Elementen in Summe 0,5 - 10,0 Masse-%, insbesondere 0,5 - 6 Masse-% oder 0,5 - 1 Masse-%, betragen.

Da Gefüge eines erfindungsgemäßen Stahl ist aufgrund der erfindungsgemäß vorgegebenen Stahllegierung im technischen Sinne vollständig austenitisch. Erfindungsgemäßer Stahl ist daher ebenso sicher amagnetisch. Er zeichnet sich dementsprechend durch eine mittels eines Permeabilitätsmessgeräts in Anlehnung an ASTM A342 und EN 60404-15 bestimmte relative Permeabilitätszahl $\mu$R aus, für die gilt: $1,0 \leq \mu R \leq 1,2$.

Erfindungsgemäße Stähle können für die Verarbeitung im Dentallabor oder für eine im größeren Maßstab industrielle Verarbeitung als Halbzeuge in Form von Gussingots oder Gussnuggets zur Verfügung gestellt werden, die bei der Weiterverarbeitung erneut erschmolzen und gießtechnisch zu Implantaten, Prothesen oder medizinischen Instrumenten und desgleichen geformt werden, die zum Einsatz im oder am menschlichen oder tierischen Körper bestimmt sind. Ihre besonders gute Vergießbarkeit macht erfindungsgemäße Stähle für diesen Fertigungsweg besonders geeignet.

Ebenso können die erfindungsgemäßen Stähle in an sich bekannter Weise zu Stahlpulvern verarbeitet werden, die anschließend mittels eines additiven Verfahrens, auch metallischer "3D-Druck" genannt, zu Implantaten, Prothesen oder im Bereich der Behandlung und Untersuchung von Mensch oder Tier eingesetzten Instrumenten geformt werden. Hierzu in Frage kommende Verfahren sind im VDI Statusreport "Additive Fertigungsverfahren", September 2014, herausgegeben vom Verein Deutscher Ingenieure e.V., Fachbereich Produktionstechnik und Fertigungsverfahren, www.vdi.de/statusadditiv und in den VDI-Richtlinien 3404 und 3405 erläutert.

Des Weiteren kann erfindungsgemäßer Stahl in Form von sogenannten Frässcheiben bereitgestellt werden, aus denen anschließend durch spanabhebende Verfahren, insbesondere Fräsen, beispielsweise durch Anwendung von bekannten CAD/CAM-Verfahren Implantate, Prothesen oder medizinische Instrumente und desgleichen hergestellt werden.

Unabhängig vom jeweiligen Verarbeitungsweg eignen sich erfindungsgemäße Stähle insbesondere zur Herstellung von Elementen, die als Zahnersatz dienen oder als Teile von Zahnersatz benötigt werden.

Dabei können aus erfindungsgemäßen Stählen erzeugte Halbzeuge, Implantate, Prothesen und desgleichen insbesondere auch einer mechanischen Feinbearbeitung unterzogen werden, die sich selbstverständlich an das jeweilige der voranstehend erläuterten formgebenden Verfahren anschließen kann.

Nachfolgend wird die Erfindung anhand einer ein Ausführungsbeispiel darstellenden Zeichnung näher erläutert. Es zeigen jeweils schematisch:

In einer ersten Versuchsreihe wurden vier Stähle M1_1 bis M1_4 erschmolzen, deren Zusammensetzungen in Tabelle 1 angegeben sind.

In einer ersten Versuchsreihe wurden vier Stähle M2_1 bis M2_5 erschmolzen, deren Zusammensetzungen in Tabelle 2 angegeben sind.

Alle nicht in den Tabellen 1 und 2 angegebenen Gehalte an Legierungselementen lagen im Verunreinigungsbereich und betrugen in Summe weniger als 0,5 Masse-%. Die Gehalte an den in den Tabellen 1 und 2 nicht aufgeführten Legierungsbestandteilen waren damit so gering, dass sie keinen Einfluss auf die Eigenschaften der Stähle M1_1 bis M1_4 und M2_1 bis M2_5 hatten.

Die Schmelzen der Stähle M1_1 bis M1_4 und M2_1 bis M2_5 sind jeweils zu Ingots vergossen worden, die das Ausgangsmaterial für die weitere Verarbeitung bildeten. Jede der Stahlschmelzen ist im technischen Sinne vollständig austenitisch erstarrt.

Zur Bewertung der magnetischen Eigenschaften der erhaltenen Ingots ist die relative Permeabilitätszahl $\mu$R der aus den Schmelzen M1_1 bis M1_4 bestehenden Ingots bestimmt worden. Die jeweils ermittelten Permeabilitätszahlen $\mu$R sind in Tabelle 1 ebenfalls verzeichnet. Es zeigte sich, dass alle aus den Stählen M1_1 bis M1_4 bestehenden Ingots zuverlässig amagnetisch waren ($\mu$R = 1).

Auch die aus den Stählen M2_1 bis M2_5 bestehenden Ingots erwiesen sich als vollständig amagnetisch.

Für die Stähle M2_1 bis M2_5 sind die Wärmeausdehnungskoeffizienten WAK bestimmt worden. Die WAK-Werte sind für die aus den Stählen M2_1 bis M2_5 bestehenden Ingots in Tabelle 2 ebenfalls verzeichnet.

Ebenso ist gemäß DIN EN ISO 22674 die Festigkeit der Ingots untersucht worden. Hier haben die aus den Stählen M1_1 bis M1_4 sowie M2_1 bis M2_5 bestehenden Ingots jeweils zuverlässig die Anforderungen der Klasse 3 erfüllt.

Im Dentallabor sind die aus den Stählen M1_1 bis M1_4 sowie M2_1 bis M2_5 jeweils bestehenden Ingots im Schmelzenintervall von 1270 - 1350 °C erneut aufgeschmolzen und in Formen zu Implantaten vergossen worden. Jeder der Stähle M1_1 bis M1_4 sowie M2_1 bis M2_5 zeigte ein sehr gutes Gießverhalten. Die Gießversuche ergaben jeweils eine vollständige Formfüllung selbst bei komplexen Geometrien.

Anschließend sind die bei den Gießversuchen erhaltenen Implantate mit einer keramischen Verblendung versehen worden. Dabei kam die von der Wegold Edelmetalle GmbH, 90350 Wendelstein, Deutschland unter der Bezeichnung "classica· Opaquer Paste · D2 · 6g" angebotene Verblendkeramik zum Einsatz, die auf einer Leucit-Glaskeramik beruht (siehe die von der Herstellerin publizierte "Classica Verarbeitungsanleitung", Druckvermerk QMF 4.5-1277, Rev. b vom 12.12.2019, die unter der URL https://www.wegold.de/?option= com edocman&task=document.viewdoc&id=738 (Auffindedatum 8. Dezember 2020) zum Download bereitsteht).

Die Verblendung der Implantate ließ sich bei jedem der aus den Stählen M1_1 bis M1_4 sowie M2_1 bis M2_5 erzeugten Implantate problemlos vornehmen und zuverlässig reproduzieren. Es traten auch unter Belastung keine Risse oder ähnliche Defekte auf.

Tabelle 1

| Stahl | C+N | C | N | Si | Mn | Cr | Mo | Ni | μ | PREN |
|-------|-----|---|---|----|----|----|----|----|----|------|
|       | [Masse-%] *) | | | | | | | | | |
| M1_1 | 1,0 | 0,4 | 0,6 | <0,1 | 21 | 18,0 | 2,0 | <0,1 | 1,0009 | 34,2 |
| M1_2 | 1,35 | 0,34 | 1,01 | <0,2 | 18,1 | 23,3 | 2,0 | <0,1 | 1,0016 | 46,6 |
| M1_3 | 1,32 | 0,34 | 0,97 | 0,2 | 18,1 | 24,3 | 1,95 | <0,1 | 1,0029 | 46,3 |
| M1_4 | 1,1 | 0,1 | 1,0 | <0,2 | 23,5 | 20,5 | 1,0 | <0,1 | 1,0009 | 39,8 |
| *) Rest Eisen und unvermeidbare Verunreinigungen | | | | | | | | | | |

Tabelle 2

| Stahl | C+N | C | N | Si | Mn | Cr | Mo | Ni | W | WAK |
|-------|-----|---|---|----|----|----|----|----|----|-----|
|       | [Masse-%] *) | | | | | | | | | $[10^{-6}/K]$ |
| M2_1 | 1,0 | 0,4 | 0,6 | <0,1 | 21 | 18,0 | 2,0 | <0,1 | - | |
| M2_2 | 1,0 | 0,4 | 0,6 | <0,1 | 21 | 18,0 | 2,0 | <0,1 | 1,0 | 17,2 |
| M2_3 | 1,0 | 0,4 | 0,6 | <0,1 | 21 | 18,0 | 2,0 | <0,1 | 2,0 | 16,8 |
| M2 4 | 1,0 | 0,4 | 0,6 | <0,1 | 21 | 18,0 | 2,0 | <0,1 | 3,0 | 16,5 |
| M2_5 | 1,0 | 0,4 | 0,6 | <0,1 | 21 | 18,0 | 2,0 | <0,1 | 4,0 | 16,3 |
| *) Rest Eisen und unvermeidbare Verunreinigungen | | | | | | | | | | |

**Patentansprüche**

1. Stahl mit einem austenitischen Gefüge, mit einer Dehngrenze Rp0,2 von mindestens 230 MPa, mit einer Bruchdehnung A von mehr als 5 % und mit einem E-Modul von mindestens 150 GPa, bestehend aus, in Masse-%,

| C: | 0,05 | - | 0,8 % , |
|----|------|---|--------|
| N: | 0,3 | - | 1,3 % , |
| Si: | | < | 2,0 % , |
| Mn: | 14 | - | 30 % , |
| Cr: | 17 | - | 27 % , |
| Mo: | 0 | - | 6 % , |
| W: | 0 | - | 6 % , |
| Nb: | 0 | - | 6 % , |
| Ga: | 0 | - | 6 % , |
| Ta | 0 | - | 6 % , |

Rest Eisen und unvermeidbaren Verunreinigungen, wobei der Gehalt an Verunreinigungen auf weniger als 0,5 % beschränkt ist und zu den Verunreinigungen weniger als 0,1 % Ni und weniger als 0,1 % Co zählen.

2. Stahl nach Anspruch 1, **dadurch gekennzeichnet, dass** der Gehalt mindestens eines Elements der Gruppe "Nb, Mo, W, Ga, Ta" mindestens 0,5 Masse-% beträgt.

3. Stahl nach einem der voranstehenden Ansprüche, **dadurch gekennzeichnet, dass** die Summe der Gehalte an den jeweils optional vorhandenen Elementen aus der Gruppe "Nb, Mo, W, Ga, Ta" - höchstens 10 Masse-% beträgt.

4. Stahl nach einem der voranstehenden Ansprüche, **dadurch gekennzeichnet, dass** für die Summe seiner Gehalte %C an C und %N an N gilt:

$$0{,}30\ \% \leq \%C + \%N \leq 2{,}0\ \%$$

5. Stahl nach einem der voranstehenden Ansprüche, **dadurch gekennzeichnet, dass** sein Mn-Gehalt mindestens 16 Masse-% beträgt.

6. Stahl nach einem der voranstehenden Ansprüche, **dadurch gekennzeichnet, dass** sein Mn-Gehalt höchstens 28 Masse-% beträgt.

7. Stahl nach einem der voranstehenden Ansprüche, **dadurch gekennzeichnet, dass** sein Cr-Gehalt mindestens 17 Masse-% beträgt.

8. Stahl nach einem der voranstehenden Ansprüche, **dadurch gekennzeichnet, dass** sein Cr-Gehalt höchstens 27 Masse-% beträgt.

9. Stahl nach einem der voranstehenden Ansprüche, **dadurch gekennzeichnet, dass** für seinen gemäß folgender Gleichung

$$PREN = \%Cr + 3{,}3 \times \%Mo + 20 \times \%N$$

aus seinem Cr-Gehalt %Cr, seinem Mo-Gehalt %Mo und seinem N-Gehalt %N, jeweils eingesetzt in Masse-%, berechneten PREN-Wert gilt:

$$25\ \% \leq PREN \leq 75\ \%$$

10. Stahl nach einem der voranstehenden Ansprüche, **dadurch gekennzeichnet, dass** für seinen Wärmeausdehnungskoeffizient WAK im Temperaturbereich von der Raumtemperatur bis 400 °C gilt:

$$WAK \leq 25 \times 10^{-6}/K$$

11. Stahl nach einem der voranstehenden Ansprüche, **dadurch gekennzeichnet, dass** für seine Permeabilitätszahl $\mu R$ gilt:

$$1{,}0 \leq \mu R \leq 1{,}2.$$

12. Verwendung eines gemäß einem der Ansprüche 1 - 11 legierten Stahls für die Herstellung von Bauteilen zur Verwendung im Mundraum von Menschen oder Tieren.

Europäisches Patentamt
European Patent Office
Office européen des brevets

# EUROPÄISCHER RECHERCHENBERICHT

Nummer der Anmeldung

EP 20 21 5553

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 104 878 316 A (UNIV NANJING SCIENCE & TECH) 2. September 2015 (2015-09-02) * Absätze [0024] - [0026]; Beispiel 1; Tabellen 1-2 * ----- | 1-12 | INV. A61K6/84 C22C1/02 C22C38/00 C22C38/02 |
| X | JP 2000 239799 A (DAIDO STEEL CO LTD) 5. September 2000 (2000-09-05) * Absätze [0017] - [0025]; Beispiel 8 * ----- | 1-12 | C22C38/22 C22C38/26 C22C38/38 |
| X | CN 105 925 814 A (UNIV NORTHEASTERN) 7. September 2016 (2016-09-07) * Absätze [0040] - [0047]; Beispiel 2 * ----- | 1-12 | |
| X | JP 2016 102244 A (JAPAN STEEL WORKS LTD:THE) 2. Juni 2016 (2016-06-02) * Absätze [0029] - [0042]; Beispiel 1; Tabelle 1 * ----- | 1-12 | |
| X | US 2011/008714 A1 (ABD ELHAMID MAHMOUD H [US] ET AL) 13. Januar 2011 (2011-01-13) * Absätze [0028], [0029]; Beispiel GM3; Tabelle 1 * ----- | 1,3-12 | |

**RECHERCHIERTE SACHGEBIETE (IPC)**

A61K
C22C

Der vorliegende Recherchenbericht wurde für alle Patentansprüche erstellt

| Recherchenort | Abschlußdatum der Recherche | Prüfer |
|---|---|---|
| Den Haag | 17. Mai 2021 | Barenbrug-van Druten |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...............................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 20 21 5553

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-05-2021

| Im Recherchenbericht angeführtes Patentdokument | Datum der Veröffentlichung | Mitglied(er) der Patentfamilie | Datum der Veröffentlichung |
|---|---|---|---|
| CN 104878316 A | 02-09-2015 | KEINE | |
| JP 2000239799 A | 05-09-2000 | KEINE | |
| CN 105925814 A | 07-09-2016 | KEINE | |
| JP 2016102244 A | 02-06-2016 | JP 6146780 B2<br>JP 2016102244 A | 14-06-2017<br>02-06-2016 |
| US 2011008714 A1 | 13-01-2011 | CN 101950807 A<br>DE 102010026330 A1<br>US 2011008714 A1 | 19-01-2011<br>07-04-2011<br>13-01-2011 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0918099 A1

- DE 10146616 A1

**In der Beschreibung aufgeführte Nicht-Patentliteratur**

- Classica Verarbeitungsanleitung. *Druckvermerk QMF 4.5-1277,* 12. Dezember 2019, https://www.we-gold.de/?option= com edocman&task=docu-ment.viewdoc&id=738